# EUROPÄISCHE PATENTSCHRIFT

(11) **EP 1 726 594 B1**
(45) Veröffentlichungstag und Bekanntmachung des Hinweises auf die Patenterteilung: **25.07.2007**
(21) Anmeldenummer: 06016495.1
(22) Anmeldetag: 15.01.2004
(51) Int. Cl.: C07F 5/02

(54) **Salze mit Cyanoborat-Anionen**
Salts comprising cyanoborate anions
Sels a base d`anions cyanoborates

(30) Priorität: 14.02.2003 DE 10306617
(43) Veröffentlichungstag der Anmeldung: 29.11.2006
(62) Teilanmeldung aus: 04702317.1
(73) Patentinhaber: Merck Patent GmbH, 64293 Darmstadt (DE)
(72) Erfinder: Welz-Biermann Urs Dr., 64646 Heppenheim (DE); Ignatiev, Nikolai Dr., 47058 Duisburg (DE); Bernhardt, Eduard Dr., 42107 Wuppertal (DE); Finze, Maik Dr., 31582 Nienburg (DE); Willner, Helge Professor Dr., 45481 Muelheim/Ruhr (DE)

(56) Entgegenhaltungen:
- WILLIAMS, DARRICK ET AL: "Synthesis of LiBC4N4, BC3N3, and Related C-N Compounds of Boron: New Precursors to Light Element Ceramics" JOURNAL OF THE AMERICAN CHEMICAL SOCIETY , 122(32), 7735-7741 CODEN: JACSAT; ISSN: 0002-7863, 2000, XP002278152
- BERNHARDT, E. ET AL: 'The tetracyanoborates M[B(CN)4], M = [Bu4N]+, Ag+, K+' ZEITSCHRIFT FUER ANORGANISCHE UND ALLGEMEINE CHEMIE Bd. 626, Nr. 2, 2000, Seiten 560 - 568, XP009030010
- BERNHARDT, E. ET AL: 'An efficient synthesis for tetracyanoborates by sinter processes' ZEITSCHRIFT FUER ANORGANISCHE UND ALLGEMEINE CHEMIE Bd. 629, Nr. 7-8, 2003, Seiten 1229 - 1234, XP009030008

## Beschreibung

Die vorliegende Erfindung betrifft ein Verfahren zur Herstellung von Alkalimetallcyanoboraten.

lonische Flüssigkeiten oder flüssige Salze sind ionische Spezies, die aus einem organischen Kation und einem i.d.R. anorganischen Anion bestehen. Sie enthalten keine neutralen Moleküle, und weisen in der Regel Schmelzpunkte kleiner 373 K auf. Im Stand der Technik sind eine Vielzahl von Verbindungen bekannt, die als ionische Flüssigkeiten Verwendung finden. Insbesondere sind sie auch Gegenstand einer Reihe von Patenten bzw. Patentanmeldungen.

So wurden lösungsmittelfreie ionische Flüssigkeiten erstmals von Hurley und Wier in einer Reihe von US-Patenten (US 2,446,331, US 2,446,339 und US 2,446,350) offenbart. Diese "bei Raumtemperatur geschmolzenen Salze" enthielten AlCl₃ und eine Vielzahl von n-Alkylpyridinium-Halogeniden.

In den letzten Jahren wurden einige Übersichtsartikel zu diesem Thema veröffentlicht (R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083; R. Hagiwara, Ya. Ito "Room temperature ionic liquids of alkylimidazolium cations and fluoroanions", Journal of Fluorine Chem., 105 (2000), 221-227).

Die Eigenschaften ionischer Flüssigkeiten, z.B. Schmelzpunkt, thermische und elektrochemische Stabilität, Viskosität, werden stark von der Natur des Anions beeinflusst. Demgegenüber können die Polarität und die Hydrophilie bzw. Lipophilie durch die geeignete Wahl des Kation/Anion-Paares variiert werden. Daher besteht grundsätzlicher Bedarf an neuen ionischen Flüssigkeiten mit variierten Eigenschaften, die zusätzliche Möglichkeiten hinsichtlich ihrer Verwendung ermöglichen.

Entscheidende Fortschritte auf dem Gebiet der ionischen Flüssigkeiten wurden mit der Entdeckung des 1-Ethyl-3-methylimidazolium Chloroaluminats erreicht. Dieses Salz hat einen breiten Flüssigbereich und ein elektrochemisches Fenster von mehr als 3 V und ist somit von großem Interesse für elektrochemische und synthetische Zwecke. Seine Anwendung ist aber durch die chemische Instabilität, vor allem gegen Feuchtigkeit, begrenzt. Nach der Entdeckung des hydrolysestabileren 1-Ethyl-3-methylimidazolium Tetrafluorborats wurden Kombinationen von Alkylimidazolium-Kationen mit anorganischen oder organischen Anionen untersucht, von denen das 1-Ethyl-3-methylimidazolium Tetrafluorborat am besten charakterisiert ist.

Die Stabilität des Imidazolium-Kations ist relativ hoch und seine Zersetzungstemperatur wird im wesentlichen durch das Anion bestimmt. So sind die 1-Ethyl-3-methylimidazolium-Salze mit Triflat- und Bis(trifluormethylsulfonyl)imid-Anionen bis 400°C stabil, wohingegen das 1-Ethyl-3-methylimidazolium Tetrafluorborat nur bis 300°C stabil ist.

Im Stand der Technik sind Borat-Anionen beschrieben, bei denen Fluor-Liganden durch Cyanid (E. Bernhardt, G. Henkel, H. Willner, Z. Anorg. Allg. Chem. 626 (2000) 560; D. Williams, B. Pleune, J. Kouvetakis, M. D. Williams, R. A. Andersen, J. Amer. Chem. Soc. 122 (2000) 7735; E. Bernhardt, M. Berkei, M. Schürmann, H. Willner, Z. Anorg. Allg. Chem. 628 (2002) 1734) und Trifluormethyl-Liganden (E. Bernhardt, G. Henkel, H. Willner, G. Pawelke, H. Bürger, Chem. Eur. J. 7 (2001) 4696; G. Pawelke, H. Bürger, Coord. Chem. Rev. 215 (2001) 243) ausgetauscht sind. Dabei werden die Trifluormethyl-Borate ausgehend von den Cyanoboraten synthetisiert, wobei allerdings die Cyanoborate schwer und nur in geringen Mengen zugänglich sind. Die Synthese von [B(CN₄)]⁻ ist arbeitsintensiv und nur in kleinem präparativem Maßstab durchführbar. Darüber hinaus sind die Ausgangsmaterialien teuer.

Aufgabe der vorliegenden Erfindung ist es ein effektives und wirtschaftlich sinnvolles Verfahren zur Herstellung dieser Borat-Salze und ihrer Vorläufer zur Verfügung zu stellen.

Diese Aufgabe wird erfindungsgemäß durch die kennzeichnenden Merkmale des Hauptanspruchs und der nebengeordneten Ansprüche gelöst.

Ein erster Gegenstand der vorliegenden Erfindung ist daher ein Verfahren zur Herstellung von Alkalimetallcyanoboraten der allgemeinen Formel (1)

M⁺ [B(CN)₄] ⁻ (1),

wobei M ausgewählt ist aus der Gruppe Li, Na, K, Rb und Cs,
bei dem die leicht zugänglichen Ausgangssubstanzen Alkalimetalltetrafluoroborat M[BF₄] (M = Li, Na, K, Rb, Cs) und Alkalimetallcyanid MCN (M = Li, Na, K, Rb, Cs) in einer Festkörperreaktion miteinander umgesetzt werden.

Vorzugsweise werden erfindungsgemäß als Alkalimetalltetrafluoroborat Kaliumtetrafluoroborat K[BF₄] oder Natriumtetrafluoroborat Na[BF₄] und als Alkalimetallcyanid Kaliumcyanid KCN oder Natriumcyanid NaCN verwendet.

In einer bevorzugten Variante des erfindungsgemäßen Verfahrens wird das Alkalimetalltetrafluoroborat mit dem Alkalimetallcyanid in Gegenwart eines Lithiumhalogenids umgesetzt. Das Lithiumhalogenid ist dabei ausgewählt aus LiCl, LiBr und Lil, besonders bevorzugt ist es Lithiumchlorid LiCl.

Alkalimetallcyanid und Lithiumhalogenid können jeweils im Überschuss von einem der beiden Reagenzien eingesetzt werden. Bevorzugt werden jedoch das Alkalimetallcyanid und das Lithiumhalogenid in etwa im molaren Verhältnis 1:1 zur Reaktion gebracht.

Das Alkalimetalltetrafluoroborat und das Alkalimetallcyanid werden vorzugsweise im molaren Verhältnis von 1:4 bis 1:12, besonders bevorzugt im molaren Verhältnis von etwa 1:9, eingesetzt.

Ganz besonders bevorzugt wird daher das molare Verhältnis Alkalimetalltetrafluoroborat : Alkalimetallcyanid : Lithiumhalogenid von ca. 1:9:9 verwendet.

Besonders bevorzugt werden als Edukte für die erfindungsgemäße Reaktion als Alkalimetalltetrafluoroborat das Kaliumtetrafluoroborat K[BF₄] und als Alkalimetallcyanid das Kaliumcyanid KCN verwendet.

Die erfindungsgemäße Festkörperreaktion wird bei Temperaturen zwischen 100°C und 500°C durchgeführt. Bevorzugt sind Temperaturen von 250 bis 400°C, besonders bevorzugt 280 - 340°C.

Ohne Einschränkung der Allgemeinheit wird an einem allgemeinen Beispiel der erfindungsgemäße Gegenstand der Festkörperreaktion erläutert:
K[BF₄], KCN und LiCl werden im molaren Verhältnis von 1:9:9 vermengt und anschließend in der Schmelze zur Reaktion gebracht werden. Die Reaktionstemperatur wird so gewählt, dass einerseits das KCN-LiCl-Gemisch ein bei 270 - 290°C schmelzendes Eutektikum bildet und sich andererseits die gebildeten Salze des Tetracyanoborats nur langsam zersetzen (< 400 - 500°C). Durch die Auswertung von Pulverdiffraktogrammen der abgekühlten Schmelze von KCN mit LiCl (molares Verhältnis 1:1) können Mischkristalle des Typs K(Cl,CN) (a = 6.34 Å, F m3m) und eine weitere nicht identifizierte Verbindung (d = 4.958, 2.878, 2.728, 2.482, 2.175 Å) nachgewiesen werden. Die Ausbeute von K[B(CN)₄] ist im Bereich von 280 - 340°C kaum temperaturabhängig und beträgt ca. 40 - 60% bezogen auf K[BF₄]. Es zeigt sich in weiteren Versuchen, dass eine Verringerung des molaren Verhältnisses von K[BF₄] zu KCN/LiCl von 1:9 auf 1:4,5 zu Ausbeuterückgängen führt. Die Raman-Spektren der Reaktionsgemische zeigen, dass das Tetracyanoborat nach der Reaktion in Form des Lithium-Salzes vorliegt (ν(CN) =2263 cm⁻¹).

Bei der analogen Reaktion unter Verwendung eines NaCN/LiCl Gemisches bilden sich in der Schmelze von NaCN mit LiCl (molares Verhältnis 1:1) Mischkristalle vom Typ (Li,Na)(Cl,CN) (a = 5.50Å Fm3m) neben wenig LiCN (d = 5.216, 3.626 Å, Smp. 160°C). Zwischen NaCN mit LiCl bildet sich im Gegensatz zu KCN/LiCl ein Eutektikum (120-140°C), die Mischkristalle schmelzen aber erst bei 360 - 540°C; dies dürfte die Ursache für die geringeren Ausbeuten (ca. 25%) an Na[B(CN)₄] sein.

Bei der Aufarbeitung der Reaktionsprodukte muss zuerst das überschüssige Cyanid zerstört werden. Es zeigt sich, dass die Oxidation des Cyanids mit wässriger 30%-iger H₂O₂-Lösung die beste Aufarbeitungsmethode ist. Die geringe Salzlast und der vollständige und schnelle Abbau des im Reaktionsgemisch verbliebenen Cyanids, sowie die guten Ausbeuten überwiegen den einzigen Nachteil, die oft heftige und schwer kontrollierbare Umsetzung des Cyanids. Das Tetracyanoborat wird anschließend aus der wässrigen Lösung extrahiert und durch Reextraktion in das K- oder Na-Salz umgewandelt.

Als alternative Methode zur Aufarbeitung der Festkörperreaktionsprodukte bietet sich die Oxidation des nicht umgesetzten Cyanids mit wässriger NaOCl-Lösung an, die innerhalb weniger Minuten unter sehr milden Bedingungen abläuft, d.h. ohne Erwärmung oder Schäumen des Reaktionsgemisches. Die Aufarbeitung erfolgt dann analog zu der mit H₂O₂. Allerdings gestaltet sich diese weitere Aufarbeitung durch die größere Salzlast arbeits- und zeitaufwendiger.

Die NMR-Spektren wurden an Lösungen in deuterierten Lösungsmitteln bei 20°C an einem Bruker Avance DRX-300 Spektrometer mit einem 5 mm Breitbandkopf ¹H/BB mit Deuterium Lock gemessen. Die Messfrequenzen der verschiedenen Kerne sind: ¹H: 300,13 MHz, ¹¹B: 96,92 MHz, ¹³C: 75,47 MHz, ¹⁹F: 282,41 MHz und ¹⁵N: 30,41 MHz. Die Methode der Referenzierung wird bei jedem Spektrum bzw. bei jedem Datensatz separat angegeben.

DSC-Messungen wurden an einem Netzsch DSC 204 Gerät durchgeführt. Zur Kalibrierung der Temperatur und der Empfindlichkeit fanden Naphthalin, Benzoesäure, KNO₃, AgNO₃, LiNO₃ und CsCl Verwendung. Von den Substanzen wurden jeweils 5-20 mg in einen Aluminium-Tiegel eingewogen und mit Aluminium-Kappen mit einer kleinen Öffnung verschlossen. Die Untersuchung erfolgte im Temperaturbereich von 25 bis 500°C. Sofern nicht anders angegeben beträgt die Aufheizrate 10 Kmin⁻¹. Während der Messung wurde der Probenraum mit trockenem Stickstoff gespült. Die Proben luftempfindlicher Substanzen wurden in einer Trockenbox präpariert und in einem mit Argon gefüllten Gläschen zum Analysengerät transportiert. Die Datenauswertung wurde mit dem Programm Netzsch Protens 4.0 vorgenommen.

Die Elementaranalysen erfolgten nach den Verbrennungsmethoden der Mikroanalytik mit einem Euro EA3000 der Firma HEKA-Tech GmbH. Die Proben luftempfindlicher Substanzen wurden in einer Trockenbox präpariert und in einem mit Argon gefüllten Gläschen zum Analysengerät transportiert. Die Fehlergrenzen für die registrierten Atome betragen: C: ±0,3 %, H: ±0,1 %, N: ±0,2 %.

### Beispiel 1: Synthese von K[B(CN)₄]

KCN, LiCl und K[BF₄] werden in einer Trockenbox (MBraun, München) grob gemörsert und miteinander vermengt. Das Gemenge wird mit einer handelsüblichen Kaffeemühle fein zermahlen. Anschließend wird das Reaktionsgemisch in einen Nickel-Tiegel (∅_{Innen} = 101 mm, *d*_{Wand} = 2 mm, h = 85 mm) überführt. Der Tiegel wird mit einem Eisen-Deckel lose abgedeckt, aus der Trockenbox in einen Muffelofen (VMK 93, Kontron Material und Strukturanalyse GmbH) überführt und erhitzt. Nach beendeter Reaktion wird der Tiegel mit der Metallabdeckung aus dem noch heißen Muffelofen genommen und an der Luft auf Raumtemperatur abgekühlt.
Das erkaltete grau-schwarze poröse Reaktionsgemisch wird aus dem Tiegel in einen Mörser überführt und grob zerstoßen. Anschließend wird der zerkleinerte Feststoff in einem 3 L Becherglas mit 150 mL Wasser versetzt und in ca. 30 mL-Portionen werden in einem Zeitraum von einer halben Stunde insgesamt 350 mL H₂O₂ (30 %-ige wässrige Lösung, ca. 3 mol) unter ständigem Rühren zugegeben. Die exotherm unter starker Gasentwicklung einsetzende Reaktion wird durch Zugabe von Eis kontrolliert. Das Reaktionsgemisch (V = 2,3 L) wird auf zwei 3 L Bechergläser aufgeteilt und mit konzentrierter HCl (ca. 300 mL, ca. 3.6 mol) angesäuert (pH 5 - 7) bis keine Gasentwicklung mehr zu beobachten ist. Anschließend wird überprüft, ob noch Cyanid-Reste im Gemisch vorhanden sind (Cyanid-Test, Merck KGaA, Darmstadt, Deutschland). Danach wird filtriert und die gelbe Lösung mit 28 mL (0.34 mol) konz. HCl unter Rühren versetzt. Anschließend erfolgt die Zugabe von 47 g (63 mL, 0.33 mol) Tripropylamin. Nach 15 Minuten Rühren wird das Reaktionsgemisch mit Dichlormethan (250, 150 und 50 mL) extrahiert. Die vereinigten organischen Phasen werden mit 200 mL H₂O gewaschen und das Waschwasser mit 25 mL Dichlormethan nachextrahiert. Die vereinigten Dichlormethan-Phasen werden über MgS0₄ getrocknet und durch eine Glasfritte (D4) filtriert. 35 g (0.63 mol) KOH werden in wenig Wasser gelöst und unter starkem Rühren zu der organischen Lösung gegeben. Es fällt sofort ein beiger schmieriger Stoff aus, der nach weiterem Rühren (30 min) am Gefäßboden verklumpt. Das Dichlormethan-Tripropylamin-Gemisch wird abdekantiert und das Produkt mit THF (200, 100 und 50 mL) aus dem Rückstand extrahiert. Die gesammelten THF-Phasen werden mit K₂C0₃ getrocknet und schließlich alle flüchtigen Bestandteile am Rotationsverdampfer entfernt. Das weiße Produkt wird mit Dichlormethan gewaschen, und im Vakuum bei Raumtemperatur getrocknet.

**Tabelle 1. Synthese von K[B(CN)₄]**

| Temp. | Zeit | K[BF_{4]} | | KCN | | LiCl | | K[B(CN)₄] | | Ausb. |
|---|---|---|---|---|---|---|---|---|---|---|
| °C | Std. | g | Mol | G | Mol | g | Mol | g | Mol | % |
| 300 | 1.5 | 37.2 | 0.30 | 170.3 | 2.62 | 116.1 | 2.74 | 29.2 ^{[a]} | 0.19 | 64 |
| 340 | 0.75 | 36.9 | 0.29 | 170.0 | 2.61 | 116.2 | 2.74 | 27.0 ^{[a]} | 0.18 | 60 |
| 340 | 1.25 | 36.9 | 0.29 | 169.9 | 2.61 | 115.9 | 2.74 | 26.7 ^{[a]} | 0.17 | 59 |
| 340 | 2 | 37.0 | 0.29 | 160.6 | 2.47 | 115.9 | 2.74 | 20.8 ^{[a]} | 0.14 | 46 |
| 340 | 3 | 36.7 | 0.29 | 172.5 | 2.65 | 102.8 | 2.42 | 20.3 ^{[b]} | 0.13 | 45 |
| 340 | 3 | 36.8 | 0.29 | 160.1 | 2.46 | 115.2 | 2.72 | 18.8 ^{[a]} | 0.12 | 42 |
| 340 | 3 | 36.7 | 0.29 | 180.9 | 2.78 | 104.7 | 2.46 | 17.4 ^{[a]} | 0.11 | 39 |

| | | | | | | | | | | |
|---|---|---|---|---|---|---|---|---|---|---|
| [a] Oxidation des nicht umgesetzten CN⁻ mit H₂O₂. [b] Oxidation des nicht umgesetzten CN⁻ mit NaOCl. | | | | | | | | | | |

¹³C{¹H}-NMR: δ= 123,3 ppm (q, 4C, CN), ¹Δ¹³C(^{10/11}B) = 0,0021 ppm, ¹*J*(¹¹B,¹³C) = 70,9 Hz; ¹¹B-NMR: δ = -38,6 ppm, ¹*J*(¹¹B,¹³C) = 71,2 Hz; Lösungsmittel: CD₃CN Referenzsubstanzen: ¹³C-NMR Lösungsmittelpeak (gegen TMS) und ¹¹B-NMR BF₃·Et₂O/CD₃CN als externer Standard.

Die NMR-Daten sind mit denen im Stand der Technik (E. Bernhardt, G. Henkel, H. Willner, Z. Anorg. Allg. Chem. 626 (2000) 560) identisch.

Ergebnisse der Elementaranalyse :

| | C [%] | H[%] | N [%] |
|---|---|---|---|
| theoretisch | 31.20 | - | 36.39 |
| gefunden | 31.35 | - | 35.97 |

Oberhalb von 450°C zersetzt sich laut DSC-Messungen das Salz.

### Beispiel 2: Synthese von Na[B(CN)₄]

170.3 g (2.62 Mol) KCN, 116.1 g (2.74 Mol) LiCl und 37.2 g (0.30 Mol) K[BF₄] werden abgewogen, grob zermörsert und miteinander vermengt. Die weitere Vorgehensweise entspricht der unter Beispiel 1 beschriebenen (Reaktionstemperatur 300 °C, Reaktionszeit 1.5 Std.), bis zum Erhalt des Dichlormethanextrakts.
In möglichst wenig Wasser (ca. 10-20 mL) werden 2 Äquivalente NaOH (ca. 25 g, 0.63 Mol) gelöst und unter starkem Rühren zu der organischen Lösung getropft. Sofort fällt ein beiger schmieriger Stoff aus, der nach weiterem Rühren (mindestens 30 min) am Gefäßboden verklumpt. Das Dichlormethan-Tripropylamin-Gemisch wird abdekantiert und das Produkt mit THF (200 mL, 100 mL und 50 mL) aus dem Rückstand extrahiert. Wird der beige Rückstand durch die Extraktion flüssig, kann durch vorsichtige Zugabe von Na₂CO₃ oder Na₂SO₄ seine viskose Konsistenz wiederhergestellt werden.
Die gesammelten THF-Phasen werden mit Na₂CO₃ oder Na₂SO₄ getrocknet und schließlich alle flüchtigen Bestandteile am Rotationsverdampfer entfernt. Das weiße Produkt wird mit Dichlormethan gewaschen, um Aminreste zu entfernen, und im Vakuum bei 60°C getrocknet. Ausbeute 25.3 g (62%, 0.18 Mol).

¹³C{¹H}-NMR: δ= 123,3 ppm (q, 4C, CN), ¹Δ¹³C(^{10/11}B) = 0,0021 ppm, ¹*J*(¹¹B,¹³C) = 70,9 Hz; ¹¹B-NMR: δ = -38,6 ppm, ¹*J*⁽¹¹B,¹³C) = 71,2 Hz; Lösungsmittel: CD₃CN Referenzsubstanzen: ¹³C-NMR Lösungsmittelpeak (gegen TMS) und ¹¹B-NMR BF₃-Et₂O/CD₃CN als externer Standard

Die NMR-Daten sind mit denen im Stand der Technik (E. Bernhardt, G. Henkel, H. Willner, Z. Anorg. Allg. Chem. 626 (2000) 560) identisch.

Ergebnisse der Elementaranalyse :

| | C [%] | H [%] | N [%] |
|---|---|---|---|
| theoretisch | 34.85 | - | 40.64 |
| gefunden | 34.60 | - | 40.15 |

### Beispiel 3: Lithium-Tetracyanoborat, Li[B(CN)₄]

5 g (32 mmol) K[B(CN)₄] werden in 20 ml Wasser gelöst, mit 8 mL 37%-iger Salzsäure (96 mmol) und 8 mL *ⁿ*Pr₃N (42 mmol) umgesetzt. Dieses Gemisch wird dann zweimal mit je 50 mL CH₂Cl₂ extrahiert, die organische Phase mit MgS0₄ getrocknet und mit einer Lösung von 3 g LiOH·H₂O (72 mmol) in 20 mL Wasser versetzt und eine Stunde kräftig gerührt. Alle flüchtigen Produkte werden im Vakuum entfernt. Aus dem Rückstand wird Li[B(CN)₄] mit 50 mL CH₃CN in einer Soxlett-Apparatur extrahiert. Die organische Phase wird am Rotationsverdampfer eingeengt. Das Rohprodukt wird aus Wasser umkristallisiert, mit 50 mL CH₂Cl₂ gewaschen und im Vakuum von Lösungsmittelresten befreit. Ausbeute 3.5 g (80%, 29 mmol).

Oberhalb von 470°C zersetzt sich laut DSC-Messungen das Salz.

## Patentansprüche

1. Verfahren zur Herstellung von Alkalimetallcyanoboraten der allgemeinen Formel (1)
M⁺ [B(CN)₄]⁻ (1),
wobei M ausgewählt ist aus der Gruppe Li, Na, K, Rb und Cs,
**dadurch gekennzeichnet, dass** ein Alkalimetalltetrafluoroborat M[BF₄], wobei M = Li, Na, K, Rb, Cs, mit einem Alkalimetallcyanid MCN, wobei M = Li, Na, K, Rb, Cs, in einer Festkörperreaktion umgesetzt wird.

2. Verfahren nach Anspruch 1,
**dadurch gekennzeichnet, dass** das Alkalimetalitetrafluoroborat K[BF₄] oder Na[BF₄] und dass das Alkalimetallcyanid KCN oder NaCN ist.

3. Verfahren nach Anspruch 1 oder 2,
**dadurch gekennzeichnet, dass** das Alkalimetalltetrafluoroborat mit dem Alkalimetallcyanid in Gegenwart eines Lithiumhalogenids ausgewählt aus LiCl, LiBr und Lil, bevorzugt in Gegenwart von LiCl, umgesetzt wird.

4. Verfahren nach Anspruch 3,
**dadurch gekennzeichnet, dass** das Alkalimetallcyanid und das Lithiumhalogenid im molaren Verhältnis 1:1 eingesetzt werden.

5. Verfahren nach einem oder mehreren der Ansprüche 1 bis 4,
**dadurch gekennzeichnet, dass** das Alkalimetalltetrafluoroborat und das Alkalimetallcyanid im molaren Verhältnis von 1:4 bis 1:12, bevorzugt im molaren Verhältnis 1:9, eingesetzt werden.

6. Verfahren nach einem oder mehreren der Ansprüche 1 bis 5,
**dadurch gekennzeichnet, dass** als Alkalimetalltetrafluoroborat K[BF₄] und als Alkalimetallcyanid KCN eingesetzt werden.

7. Verfahren nach einem oder mehreren der Ansprüche 1 bis 6,
**dadurch gekennzeichnet, dass** die Reaktion bei Temperaturen zwischen 100°C und 500°C, bevorzugt bei 250 - 400°C, besonders bevorzugt bei 280 - 340°C, durchgeführt wird.

## Claims

1. Process for the preparation of alkali metal cyanoborates of the general formula (1)
M⁺ [B(CN)₄]⁻ (1),
where M is selected from the group Li, Na, K, Rb and Cs,
**characterised in that** an alkali metal tetrafluoroborate M[BF₄], where M = Li, Na, K, Rb, Cs, is reacted with an alkali metal cyanide MCN, where M = Li, Na, K, Rb, Cs, in a solid-state reaction.

2. Process according to Claim 1,
**characterised in that** the alkali metal tetrafluoroborate is K[BF₄] or Na[BF₄] and **in that** the alkali metal cyanide is KCN or NaCN.

3. Process according to Claim 1 or 2,
**characterised in that** the alkali metal tetrafluoroborate is reacted with the alkali metal cyanide in the presence of a lithium halide selected from LiCl, LiBr and Lil, preferably in the presence of LiCl.

4. Process according to Claim 3,
**characterised in that** the alkali metal cyanide and the lithium halide are employed in the molar ratio 1:1.

5. Process according to one or more of Claims 1 to 4,
**characterised in that** the alkali metal tetrafluoroborate and the alkali metal cyanide are employed in the molar ratio from 1:4 to 1:12, preferably in the molar ratio 1:9.

6. Process according to one or more of Claims 1 to 5,
**characterised in that** the alkali metal tetrafluoroborate employed is K[BF₄] and the alkali metal cyanide employed is KCN.

7. Process according to one or more of Claims 1 to 6,
**characterised in that** the reaction is carried out at temperatures between 100°C and 500°C, preferably at 250 - 400°C, particularly preferably at 280 - 340°C.

## Revendications

1. Procédé de préparation de cyanoborates de métal alcalin de formule générale (1)
M⁺ [B(CN)₄]⁻ (1),
dans laquelle M est choisi parmi le groupe constitué par Li, Na, K, Rb et Cs,
**caractérisé en ce qu'**un tétrafluoroborate de métal alcalin M[BF₄], où M = Li, Na, K, Rb, Cs, est réagi avec un cyanure de métal alcalin MCN, où M = Li, Na, K, Rb, Cs, dans une réaction à l'état solide.

2. Procédé selon la revendication 1,
**caractérisé en ce que** le tétrafluoroborate de métal alcalin est K[BF₄] ou Na[BF₄] et **en ce que** le cyanure de métal alcalin est KCN ou NaCN.

3. Procédé selon la revendication 1 ou 2,
**caractérisé en ce que** le tétrafluoroborate de métal alcalin est réagi avec le cyanure de métal alcalin en présence d'un halogénure de lithium choisi parmi LiCl, LiBr et Lil, préférablement en présence de LiCl.

4. Procédé selon la revendication 3,
**caractérisé en ce que** le cyanure de métal alcalin et l'halogénure de lithium sont employés selon le rapport molaire 1:1.

5. Procédé selon l'une ou plusieurs parmi les revendications 1 à 4,
**caractérisé en ce que** le tétrafluoroborate de métal alcalin et le cyanure de métal alcalin sont employés selon le rapport molaire allant de 1:4 à 1:12, préférablement selon le rapport molaire 1:9.

6. Procédé selon l'une ou plusieurs parmi les revendications 1 à 5,
**caractérisé en ce que** le tétrafluoroborate de métal alcalin employé est K[BF₄] et le cyanure de métal alcalin employé est KCN.

7. Procédé selon l'une ou plusieurs parmi les revendications 1 à 6,
**caractérisé en ce que** la réaction est réalisée à des températures comprises entre 100°C et 500°C, préférablement à 250 - 400°C, particulièrement préférablement à 280 - 340°C.
